(19) Europäisches Patentamt
European Patent Office
Office européen des brevets

(11) **EP 1 685 354 B1**

(12) **EUROPEAN PATENT SPECIFICATION**

(45) Date of publication and mention
of the grant of the patent:
**04.03.2009 Bulletin 2009/10**

(51) Int Cl.:
*F25D 29/00* *(2006.01)*      *F25D 17/04* *(2006.01)*

(21) Application number: **03819000.5**

(86) International application number:
**PCT/TR2003/000087**

(22) Date of filing: **17.11.2003**

(87) International publication number:
**WO 2005/047788 (26.05.2005 Gazette 2005/21)**

(54) **COOLER COMPRISING GAS SENSOR**

EINEN GASSENSOR UMFASSENDE KÜHLVORRICHTUNG

REFRIGERATEUR COMPORTANT UN CAPTEUR DE GAZ

(84) Designated Contracting States:
**AT BE BG CH CY CZ DE DK EE ES FI FR GB GR HU IE IT LI LU MC NL PT RO SE SI SK TR**

(43) Date of publication of application:
**02.08.2006 Bulletin 2006/31**

(73) Proprietor: **Vestel Beyaz Esya Sanayi Ve Ticaret A.S.**
**45030 Manisa (TR)**

(72) Inventor: **Hekimoglu, Aydin**
**Bostanli**
**35545 Izmir (TR)**

(74) Representative: **Cayli, Hülya**
**Paragon Consultancy Inc.**
**Koza Sokak No: 60/6**
**GOP**
**06700 Ankara (TR)**

(56) References cited:
**EP-A- 0 509 328**         **DE-A- 19 806 041**

- **PATENT ABSTRACTS OF JAPAN vol. 2003, no. 01, 14 January 2003 (2003-01-14) & JP 2002 267338 A (MATSUSHITA REFRIG CO LTD), 18 September 2002 (2002-09-18)**
- **PATENT ABSTRACTS OF JAPAN vol. 2003, no. 07, 3 July 2003 (2003-07-03) & JP 2003 075383 A (MATSUSHITA REFRIG CO LTD), 12 March 2003 (2003-03-12)**
- **PATENT ABSTRACTS OF JAPAN vol. 2003, no. 12, 5 December 2003 (2003-12-05) & JP 2003 247773 A (MATSUSHITA REFRIG CO LTD), 5 September 2003 (2003-09-05)**

## Description

## Technical Field

This invention is related to a gas controlling method informing the user that the foodstuffs in the refrigerator started to deteriorate.

## Background of the Art

Foodstuffs have to be conserved under certain temperatures in order preserve their freshness. Refrigerators developed for this purpose are composed of various compartments. Foodstuffs are preserved in either cooler or freezer compartments according to their characteristics and to the time period required for preservation. Especially vegetables and fruits are preserved in a cooler compartment devised as a separate place.

Fruits and vegetables placed in refrigerator can preserve their freshness for a certain period of time. A gas discharge takes place from vegetables and fruits which begin to deteriorate at the end of this period. This gas discharge causes a bad smell both in the vegetable compartment and the cooler while at the same time shortening the decay period of other fresh vegetables and fruits.

In the present known situation of the technology, there are many sensors developed for detecting gas which have various operating principles according to the characteristics of the gas they detected. Usage of these sensors in refrigerators has been described by the American Patent Nr. US 5621162. This patent is related with a sensor which detects gases discharging from deteriorating foodstuffs, especially from vegetables and fruits. A sensitive sheet within the sensor described in the above mentioned patent absorbs oxygen ions and when these ions combine with sulfuric gas ions they emit electrons. As a result of this phenomenon, electric conductivity of sensitive sheet changes and the voltage difference information sent by the sensor to microprocessor activates systems like temperature adjustment, humidity control and pressure control thus satisfying the conditions of preserving and maintaining freshness of foodstuffs contained in the refrigerator.

But the above stated patented system describes no information about informing the knowledge of deteriorated fruits and vegetables to the user. In that system, user can understand the decayed foodstuffs by only senses of seeing and smelling. But the smell when foodstuffs produce as they begin to decay may not be perceived by users. As the deterioration of the foodstuffs proceeds, the smell they release increases, and only then users can understand that they are deteriorating. In other cases, users can also only understand foodstuff is deteriorating, after they take and look at the foodstuff when they needed it. Until user needs that foodstuff, it will not be understood that it has deteriorated. A gas controlling method used in a refrigerator which enables informing the user that the foodstuffs in the vegetable compartment started to deteriorate is disclosed in JP 2002267338.

## Aim of the Invention

It is related to a gas controlling method used in a refrigerator which enables informing of users with the knowledge of foodstuffs that began to deteriorate. Object of the invention is to improve the gas detection around the vegetable container and the controlling method.

## Description of Figures

Figure-1 Perspective view of the refrigerator with smell sensor.
Figure-2 The flowchart diagram of a preferred application of gas control method subject to this invention.
Figure-3 The flowchart diagram of another preferred application of gas control method subject to this invention.

The numbers of the parts depicted in the figures and their explanations are defined below:

1. Refrigerator
2. Body
3. Cooler door
4. Freezer door
5. Vegetable compartment
6. Back face
7. Right side face
8. Left Side face
9. Left vegetable container
10. Right vegetable container
11. Side gas sensor
12. Back gas sensor
13. Door gas sensor
14. Display panel
15. Visual Alert Unit
16. Audio Alert Unit

The refrigerator (1) comprises a body (2); a vegetable compartment (5) within the lower part of this body; at least one door (3); at least one gas sensor (11, 12, 13) that can detect the gas released by vegetables and fruits when they begin to deteriorate; a microprocessor and a display panel (14).

There is at least one gas sensor at the vegetable compartment (5) and at least one at cooler door (3).

In the preferred embodiment, there is at least one gas sensor (11, 12) at right side face (7), left side face (8), and back face (6) of the vegetable compartment (5) in which vegetable container (10) is placed. The distance between these sensors (11, 12) and the bottom of vegetable compartment (5) is determined as to be more than the height of vegetable containers (9, 10).

Cooler door (3) contains at least one door gas sensor (13). The distance between these sensors (13) and the bottom of door is determined as to be more than the height of vegetable container.

Sensors (11, 12, 13) contained within the vegetable compartment (5) and the door (3) of the refrigerator (1) have a feature of detecting gases that can be released as a consequence of deterioration, detecting their variation of concentration, and converting these into digital values. Gas sensors having this feature, are used for different purposes at the current known state of technology.

Knowledge about the gases released from deteriorating vegetables and fruits which are likely to be placed inside the vegetable compartment and about their concentration values have been preloaded into the microprocessor of the refrigerator (1) subject to this invention. Data detected by sensors (11, 12, 13) and converted into digital values are transmitted to microprocessor through as cable group.

Display panel (14) has on it a visual alert unit (15) and an audio alert unit (16). These units (15, .16) through electronic connection with microprocessor, operate in accordance with signals coming from it.

Refrigerator (1) comprises of preferably two doors (3, 4), two vegetable containers (9, 10), and at least six gas sensors (11, 12, 13). Vegetable compartment's right and left side faces (7, 8) have both one side gas sensor (11) and its back face (6) has two back gas sensors (12). Back gas sensors (12) are mounted on the back side (6) of vegetable compartment in a way that one of them at the back and middle of right vegetable container (10) and the other at the back and middle of left vegetable container (9). Door gas sensors (13) are mounted on the inner plastic of refrigerator door (3) horizontally in line with the back gas sensors (12).

Alert units (15, 16) of the refrigerator (1) are preferably on the display panel (14) which is mounted on freezer door (4).

With the gas control method performed by the gas sensors (11, 12, 13) of the refrigerator (1), beginning of deterioration of fruits and vegetables placed in the vegetable container is informed to the user through and alerting step. This step comprises of audio and/or visual alerting.

In one of the preferred applications of the gas control method, subject to this invention, performed by the gas sensors (11, 12, 13) of the refrigerator (1), at first, numeric values ($D_{threshold}$) of the gases released from deteriorating vegetables and fruits that are likely to be placed inside the vegetable container are loaded (100) into the memory of microprocessor.

At the first detection step (200), gases released by vegetables and fruits are detected by the gas sensors (11, 12, 13) which are in the vegetable compartment (5) and on the door (3) and then converted into converted into numeric values ($D_i$) Then this converted information is transmitted to the microprocessor on the electronic command board by data cables. Gas concentration in the vegetable compartment (5) is continuously measured by the gas sensors (11, 12, 13) and the numeric value ($D_i$) transmitted to microprocessor is updated.

In the first comparison step (300), the numeric values ($D_i$) transmitted by the gas sensors (11, 12, 13) both in the vegetable compartment (4) and on the door (3) are compared with the numeric values ($D_{threshold}$) of the gases preloaded on the microprocessor which shows the beginning of deterioration of vegetables and fruits that are likely to be placed in the vegetable container. As a result of this comparison step (300), in the case that foodstuffs in the vegetable container (5) are decided to begin to deteriorate,

$$(D_i) \geq (D_{threshold}),$$

this decision is transmitted to the visual alert unit (15) found on display panel (14).

As a result of this comparison step (300), in the case that foodstuffs in the vegetable compartment (5) are not decided to begin to deteriorate,

$$(D_i) < (D_{threshold}),$$

this procedure returns to the first step of detection (200).

As a result of first comparison step (300), in the case that foodstuffs are decided to begin to deteriorate $[(D_i) \geq (D_{threshold})]$; the method proceeds to visual alert step (400). In visual alert step (400), the light source on the display panel (14) is set to 'on' position. Also the ($D_i$) value decided as visual alert after being compared with the preloaded values ($D_{threshold}$) is saved (410) as ($D_c$) by the microprocessor.

Microprocessor's decision of visual alert is updated (500) with the values ($D_g$) received from gas sensors (11, 12, 13) after a predetermined time period ($t_g$).

After this update (500) step, the method proceeds to second comparison step (600). In the second comparison step (600), the gas concentration numeric value ($D_g$) received from gas sensors (11, 12, 13) is compared with the visual alert decision value ($D_c$) after a time period of ($t_g$).

If after the second comparison step (600), microprocessor decides that gas concentration of vegetable compartment (5) is reduced,

$$(D_g) < (D_c)$$

then the deteriorating foodstuff is assumed to be taken out of the refrigerator (1), this decision is sent to the visual alert unit (15) on the display panel (14), the light source is switched from on to off position, and the first step of detection is returned.

If at the end of the predetermined time ($t_g$), after comparing (600) gas concentration values ($D_g$) received from gas sensors (11, 12, 13) with the visual alert decision value ($D_c$), the gas concentration of the vegetable container (5) is decided to increase,

$$(D_g) \geq (D_c)$$

the deteriorating foodstuff is assumed not taken out of the refrigerator (1) yet, and decision is sent to the visual alert unit (16) on the display panel (14).

Audio alert step (700) comprises of switching the audio source located on the display panel (14) to on position as a result of this decision of microprocessor. Audio source switches back to off position, in the case that the foodstuff, which is decided by microprocessor to be deteriorating, is taken out of the refrigerator (1).

In another preferred application of the gas control method, subject to this invention, performed by this refrigerator (1), in the case that during the first comparison step (300) foodstuffs in the vegetable compartment are decided to begin to deteriorate,

$$(D_i) \geq (D_{threshold})$$

directly audio alerting step (700) is activated. This application is preferred in the case that refrigerator usage is not very frequent. Since the user can not be informed of the visual alert decision in a timely manner when the refrigerator is not used so often, the information that the foodstuffs began to deteriorate can be informed to the user through an audio alerting decision.

The gas control method, subject to this invention, ensures taking vegetables and fruits out of the refrigerator before they begin to deteriorate. Thus with this method vegetable and fruits are consumed before they deteriorate and other foodstuffs are preserved from deteriorating in a short time because of the gases released by deteriorated vegetables and fruits.

**Claims**

1. A gas controlling method used in a refrigerator that comprises a body (2), a vegetable compartment (5) at the lower part of this body, at least one vegetable container placed in the vegetable compartment (5), at least one door, at least one gas sensor mounted on the cooler door and at least one on the vegetable compartment (5) which detect the gases released by the foodstuffs in the vegetable compartment (5) and convert to digital value, a microprocessor that stores in its memory the digital data of the gases released when the vegetables and fruits start to deteriorate, a visual alert unit (15), an audio alert unit (16) that work with the signals coming from the microprocessor, said gas controlling method informing the user that the foodstuffs in the refrigerator started to deteriorate, and comprising

    i) loading the digital data ($D_{threshold}$) of gases released when the vegetables and fruits likely to be stored at the vegetable compartment started to deteriorate to the memory of the microprocessor (100),
    ii) a first detection step (200) in which the gases released by the vegetables and fruits in the vegetable compartment are detected by the gas sensors in both the vegetable compartment and the door and converted to the digital values ($D_i$),
    iii) a first comparison step (300) in which said digital data and values ($D_{threshold}$, $D_i$) are compared by the microprocessor.

whereas the method has an alert step by which the user is informed with an alerting unit when said digital values ($D_i$) are above or equal to said digital data ($D_{threshold}$) in the first comparison step (300), indicating that the foodstuffs in the vegetable compartment started to deteriorate.

2. A gas controlling method according to claim 1 wherein it returns to the first detection step (200) when said digital values ($D_i$) are below said digital data ($D_{threshold}$) in the first comparison step (300), indicating that the foodstuffs in the vegetable compartment are not deteriorated.

3. A gas controlling method according to claim 1 or 2 when the first comparison step (300) indicates that the foodstuffs in the vegetable compartment started to deteriorate (($D_i$) $\geq$ ($D_{threshold}$)), further comprising:

    i) a visual alerting step (400) where the user is warned by switching on a light source,
    ii) saving the digital value $D_i$ as $D_c$ in the memory of the microprocessor (410),
    iii) updating (500) the visual alerting decision (400) of the microprocessor with the value $D_g$ coming from the gas sensors after a predetermined time $t_g$,
    iv) a second comparison step (600) where the digital value $D_g$ of the gas concentration coming from the gas sensors after the time $t_g$ is compared to the value of visual warning decision $D_c$,
    v) an audio alerting step (700) where the user is warned by switching on the audio source when $D_g \geq D_c$ in the second comparison step (600).

4. A gas controlling method according to claim 3 wherein it returns to the first detection step (200) $D_g < D_c$ in the second comparison step (600).

5. A gas controlling method according to claim 1 or 2 **characterized by** an audio alerting step (700) where the user is warned by switching on the audio source when it the first comparison step (300) indicates that the foodstuffs in the vegetable compartment started to deteriorate (($D_i$) ≥ ($D_{threshold}$)).

**Patentansprüche**

1. Gasüberwachungsverfahren, das in einem Kühlschrank eingesetzt wird, der aufweist: einen Korpus (2), ein Gemüsefach (5) am unteren Teil dieses Korpus, mindestens einen Gemüsebehälter, der in das Gemüsefach (5) eingesetzt ist, mindestens eine Tür, mindestens einen Gassensor, der an der Kühlfachtür und mindestens einen, der am Gemüsefach (5) angebracht ist, welche die Gase erfassen, die von den Nahrungsmitteln im Gemüsefach (5) freigesetzt werden und in einen digitalen Wert umwandeln, einen Mikroprozessor, der in seinem Speicher die digitalen Werte der Gase speichert, die freigesetzt werden, wenn das Gemüse und Obst zu verderben beginnt, eine visuelle Alarmmeldungseinheit (15) und eine akustische Alarmmeldungseinheit (16), die mit den vom Mikroprozessor kommenden Signalen arbeiten, wobei das Gasüberwachungsverfahren den Benutzer informiert, dass die Nahrungsmittel im Kühlschrank zu verderben begannen, Folgendes umfassend:

i) Eingeben der digitalen Daten ($D_{Schwellenwert}$) von Gasen in den Speicher des Mikroprozessors (100), die freigesetzt werden, wenn das voraussichtlich im Gemüsefach zu lagernde Gemüse und Obst zu verderben begann,
ii) einen ersten Erfassungsschritt (200), bei dem die vom Gemüse und Obst im Gemüsefach freigesetzten Gase von den Gassensoren sowohl im Gemüsefach als auch der Tür erfasst und in digitale Werte ($D_i$) umgewandelt werden,
iii) einen ersten Vergleichsschritt (300), bei dem die digitalen Daten und Werte ($D_{Schwellenwert}$, $D_i$) durch den Mikroprozessor verglichen werden,

wobei das Verfahren einen Alarmmeldungsschritt aufweist, durch den der Benutzer mit einer Alarmmeldungseinheit informiert wird, wenn im ersten Vergleichsschritt (300) die digitalen Werte ($D_i$) über den digitalen Daten ($D_{Schwellenwert}$) liegen oder gleich diesen sind, wodurch angezeigt wird, dass die Nahrungsmittel im Gemüsedach zu verderben begannen.

2. Gasüberwachungsverfahren nach Anspruch 1, wobei es zum ersten Erfassungsschritt (200) zurückkehrt, wenn im ersten Vergleichsschritt (300) die digitalen Werte ($D_i$) unter den digitalen Daten ($D_{Schwellenwert}$) liegen, wodurch angezeigt wird, dass die Nahrungsmittel im Gemüsefach nicht verdorben sind.

3. Gasüberwachungsverfahren nach Anspruch 1 oder 2, das, wenn der erste Vergleichsschritt (300) anzeigt, dass die Nahrungsmittel im Gemüsefach zu verderben begannen (($D_i$) ≥ ($D_{Schwellenwert}$)), darüber hinaus umfasst:

i) einen visuellen Alarmmeldungsschritt (400), bei dem der Benutzer durch Einschalten einer Lichtquelle gewarnt wird,
ii) Speichern der digitalen Werte $D_i$ als $D_c$ im Speicher des Mikroprozessors (410),
iii) Aktualisieren (500) der visuellen Alarmmeldungsentscheidung (400) des Mikroprozessors mit dem von den Gassensoren kommenden Wert $D_g$ nach einer vorgestimmten Zeit $t_g$,
iv) einen zweiten Vergleichsschritt (600), bei dem der von den Gassensoren kommende digitale Wert $D_g$ der Gaskonzentration nach der Zeit tg mit dem Wert der visuellen Alarmmeldungsentscheidung $D_c$ verglichen wird,
v) einen akustischen Alarmmeldungsschritt (700), bei dem der Benutzer durch Einschalten der akustischen Quelle gewarnt wird, wenn im zweiten Vergleichsschritt (600) $D_g ≥ D_c$ ist.

4. Gasüberwachungsverfahren nach Anspruch 3, wobei es zum ersten Erfassungsschritt (200) zurückkehrt, wenn im zweiten Vergleichsschritt (600) $D_d < D_c$ ist.

5. Gasüberwachungsverfahren nach Anspruch 1 oder 2, **gekennzeichnet durch** einen akustischen Alarmmeldungsschritt (700), bei dem der Benutzer **durch** Einschalten der akustischen Quelle gewarnt wird, wenn der erste Vergleichsschritt (300) anzeigt, dass die Nahrungsmittel im Gemüsefach zu verderben begannen (($D_i$) ≥ ($D_{Schwellenwert}$)).

**Revendications**

1. Procédé de contrôle de gaz utilisé dans un réfrigérateur qui comprend un corps (2), un compartiment à légumes (5) au niveau de la partie inférieure de ce corps, au moins un bac à légumes placé dans le compartiment à légumes (5), au moins une porte, au moins un capteur de gaz monté sur la porte du refroidisseur et au moins un monté sur le compartiment à légumes (5) qui détectent les gaz libérés par les aliments dans le compartiment à légumes (5) et les convertissent en valeur numérique, un microprocesseur qui mémorise dans sa mémoire les données numériques des gaz libérés lorsque les légumes et les fruits commencent à se détériorer, une unité d'alerte visuelle (15), une unité d'alerte audio (16) qui fonctionnent avec les signaux provenant du microprocesseur, ledit procédé de contrôle de gaz informant l'utilisateur que les aliments dans le réfrigérateur ont commencé à se détériorer, et comprenant :

i) le chargement des données numériques ($D_{threshold}$) des gaz libérés lorsque les légumes et les fruits susceptibles d'être stockés dans le compartiment à légumes ont commencé à se détériorer dans la mémoire du microprocesseur (100),
ii) une première étape de détection (200) au cours de laquelle les gaz libérés par les légumes et les fruits dans le compartiment à légumes sont détectés par les capteurs de gaz à la fois dans le compartiment à légumes et sur la porte et convertis en les valeurs numériques ($D_i$),
iii) une première étape de comparaison (300) au cours de laquelle lesdites données et valeurs numériques ($D_{threshold}$, $D_i$) sont comparées par le microprocesseur,

tandis que le procédé comporte une étape d'alerte par laquelle l'utilisateur est informé par une unité d'alerte lorsque lesdites valeurs numériques ($D_i$) sont supérieures ou égales auxdites données numériques ($D_{threshold}$) au cours de la première étape de comparaison (300), indiquant que les aliments dans le compartiment à légumes ont commencé à se détériorer.

2. Procédé de contrôle de gaz selon la revendication 1, dans lequel le procédé retourne à la première étape de détection (200) lorsque lesdites valeurs numériques ($D_i$) sont inférieures auxdites données numériques ($D_{threshold}$) au cours de la première étape de comparaison (300), indiquant que les aliments dans le compartiment à légumes ne sont pas détériorés.

3. Procédé de contrôle de gaz selon la revendication 1 ou 2, lorsque la première étape de comparaison (300) indique que les aliments dans le compartiment à légumes ont commencé à se détériorer (($D_i$) ≥ ($D_{threshold}$)), comprenant en outre :

i) une étape d'alerte visuelle (400) au cours de laquelle l'utilisateur est averti par la mise en marche d'une source de lumière,
ii) la sauvegarde de la valeur numérique $D_i$ en tant que $D_c$ dans la mémoire du microprocesseur (410),
iii) la mise à jour (500) de la décision d'alerte visuelle (400) du microprocesseur avec la valeur $D_g$ provenant des capteurs de gaz après un temps prédéterminé $t_n$,
iv) une deuxième étape de comparaison (600) au cours de laquelle la valeur numérique $D_g$ de la concentration de gaz provenant des capteurs de gaz après le temps $t_g$ est comparée à la valeur de décision d'alerte visuelle $D_G$,
v) une étape d'alerte audio (700) au cours de laquelle l'utilisateur est averti par la mise en marche de la source audio lorsque $D_g \geq D_c$ au cours de la deuxième étape de comparaison (600).

4. Procédé de contrôle de gaz selon la revendication 3, dans lequel le procédé retourne à la première étape de détection (200) $D_g < D_c$ au cours de la deuxième étape de comparaison (600).

5. Procédé de contrôle de gaz selon la revendication 1 ou 2, **caractérisé par** une étape d'alerte audio (700) au cours de laquelle l'utilisateur est averti par la mise en marche de la source audio lorsque la première étape de comparaison (300) indique que les aliments dans le compartiment à légumes ont commencé à se détériorer (($D_i$) ≥ ($D_{threshold}$)).

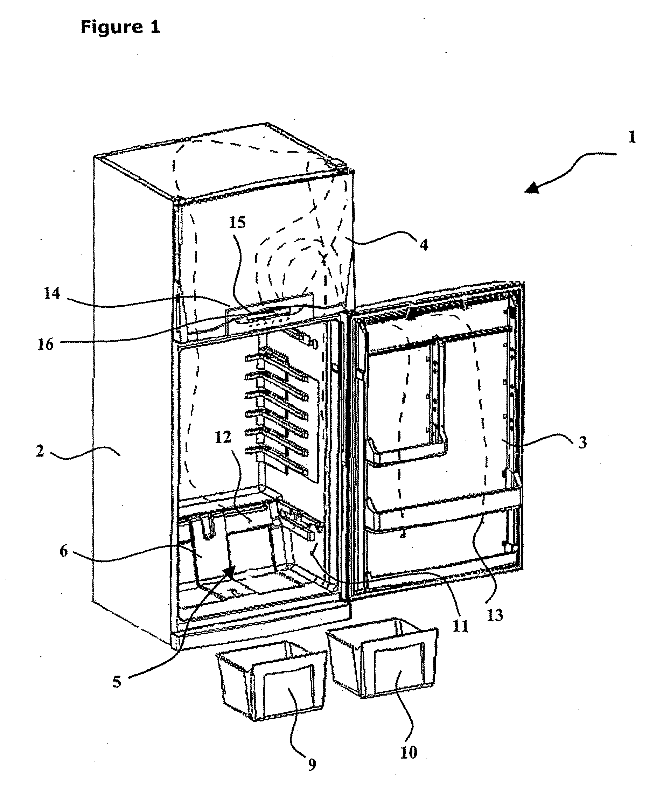

**Figure 1**

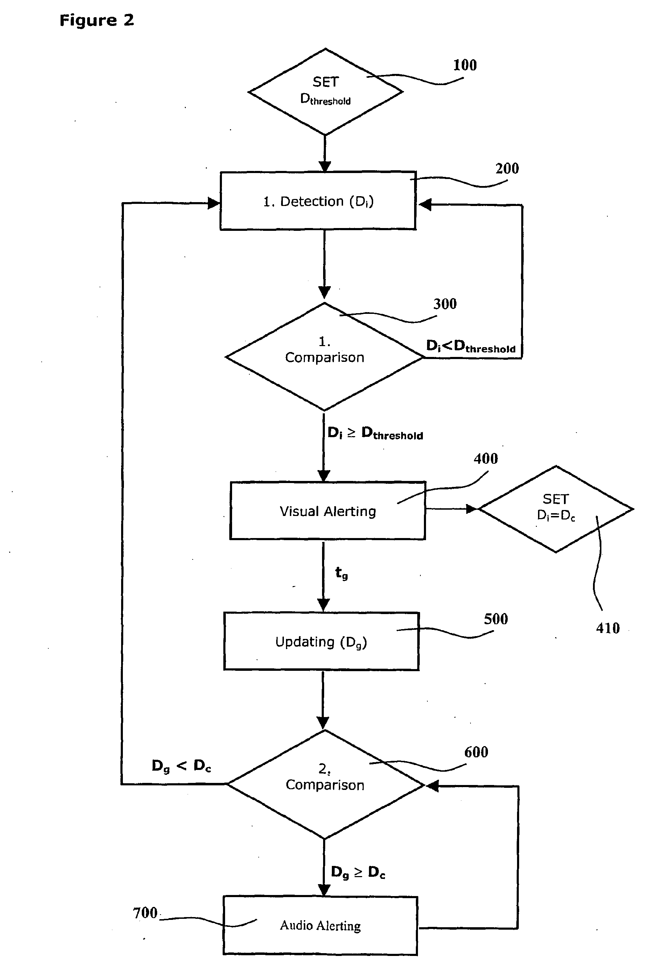

**Figure 2**

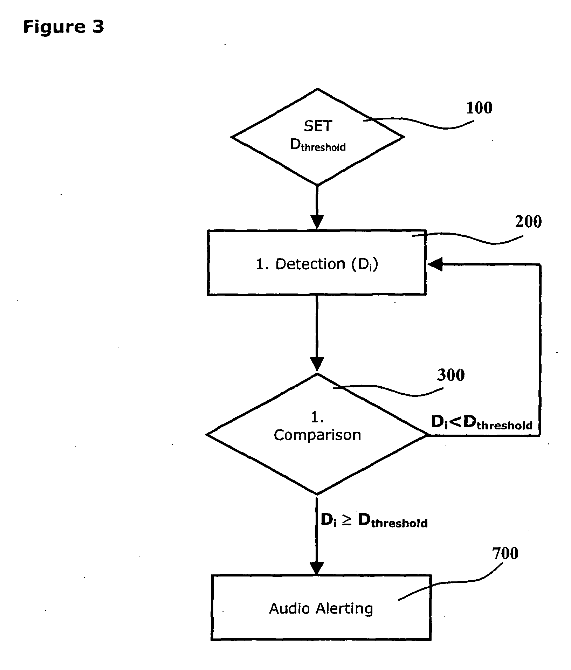

**Figure 3**

**EP 1 685 354 B1**

## REFERENCES CITED IN THE DESCRIPTION

**Patent documents cited in the description**

- US 5621162 A
- JP 2002267338 B